# EUROPEAN PATENT APPLICATION

(11) **EP 2 889 380 A1**
(43) Date of publication of application: **01.07.2015**
(21) Application number: 13199610.0
(22) Date of filing: 24.12.2013
(51) Int. Cl.: C12Q 1/68

(54) **Metagenomic analysis of samples**

(71) Applicant: Université de Liège, 4031 Angleur (BE); Quality Partner S.A., 4040 Herstal (BE)
(72) Inventor: Daube, Georges, 4000 Liège (BE); Taminiau, Bernard, 4000 Liège (BE); Nezer, Carine, 4040 Herstal (BE); Delhalle, Laurent, 4040 Herstal (BE)

(57) **Abstract**

A method of metagenomic analysis of a food or stool sample to detect and quantitate microorganisms present in said sample, the analysis comprising amplification of the V2-V3 region of bacterial 16S rRNA in said sample

SEQUENCE LISTING

<110> Université de Liege
      Université de Liege, Université of Liege

<120> Metagenomic analysis of samples

<130> JB1

<160> 83

<170> PatentIn version 3.5

<210> 1
<211> 21
<212> DNA
<213> consensus

<400> 1
gagagtttga tymtggctca g                                                21


<210> 2
<211> 17
<212> DNA
<213> consensus

<400> 2
accgcggctg ctggcac                                                     17


<210> 3
<211> 82
<212> DNA
<213> chloroplast

<400> 3
gagagttcga tcctggctca ggatgaacgc tggcggcatg cttaacacat gcaagtcgga      60

cgggaagtgg tgtttccagt gg                                               82


<210> 4
<211> 82
<212> DNA
<213> consensus

<400> 4
gagagttcga tcctggctca ggatgaacgc tggcggcatg cttaacacat gcaagtcgga      60

cgggaagtgg tgtttccagt gg                                               82


<210> 5
<211> 82
<212> DNA
<213> chloroplast

<400> 5
gagagtttga tcatggctca ggatgaacgc tggcggcatg cttaacacat gcaagtcgga      60

cgggaagtgg tgtttccagt gg                                               82

<210> 6
<211> 82
<212> DNA
<213> consensus

<400> 6
gagagtttga tcatggctca ggatgaacgc tggcggcatg cttaacacat gcaagtcgga 60

cgggaagtgg tgtttccagt gg 82


<210> 7
<211> 82
<212> DNA
<213> chloroplast

<400> 7
gagagtttga tcatggctca ggatgaacgc tggcggcatg cttaacacat gcaagtcgga 60

cgggaagtgg tgtttccagt gg 82


<210> 8
<211> 82
<212> DNA
<213> chloroplast

<400> 8
gagagtttga tcatggctca ggatgaacgc tggcggcatg cttaacacat gcaagtcgga 60

cgggaagtgg tgtttccagt gg 82


<210> 9
<211> 82
<212> DNA
<213> chloroplast

<400> 9
gagagtttga tcatggctca ggatgaacgc tggcggcatg cttaacacat gcaagtcgaa 60

cgggaagtgg tgtttccagt gg 82


<210> 10
<211> 82
<212> DNA
<213> chloroplast

<400> 10
gagagtttga tcatggctca ggatgaacgc tggcggcatg cttaacacat gcaagtcgaa 60

cgggaagtgg tgtttccagt gg 82


<210> 11
<211> 94
<212> DNA
<213> Pseudomonas

<400> 11
gagagtttga tcatggctca gattgaacgc tggcggcagg cctaacacat gtcaagtcga 60

gcggatgaga agagcttgct cttcgattca gcgg                                94

<210>  12
<211>  101
<212>  DNA
<213>  Xanthomonas

<400>  12
gagagtttga tcatggctca gagtgaacgc tggcggcagg cctaacacat gcaagtcgaa      60

cggcagcggg taggaagctt gcttcctatg ccggcgagtg g                         101

<210>  13
<211>  99
<212>  DNA
<213>  Escherichia coli

<400>  13
gagagtttga tcatggctca gattgaacgc tggcggcagg cctaacacat gcaagtcgaa      60

cggtaacagg aagcagcttg ctgtttcgct gacgagtgg                            99

<210>  14
<211>  112
<212>  DNA
<213>  Lactococcus

<400>  14
gagagtttga tcctggctca ggatgaacgc tggcggcgtg cctaatacat gcaagtcgaa      60

cgaaacttct ttatcaccga gtgcttgcac tcaccgataa agagttgagt gg             112

<210>  15
<211>  97
<212>  DNA
<213>  Brochothrix

<400>  15
gagagtttga tcctggctca ggacgaacgc tggcggcgtg cctaatacat gcaagtcgaa      60

cgaacggata aagagcttgc tcttttgaag ttagtgg                              97

<210>  16
<211>  91
<212>  DNA
<213>  Arthrobacter

<400>  16
gagagtttga tcatggctca ggatgaacgc tggcggcgtg cttaacacat gcaagtcgaa      60

cgatgaacct cacttgtggg gggattagtg g                                    91

<210>  17
<211>  17
<212>  DNA
<213>  consensus

<400>  17
gaagtggtgt ttccagt                                                    17


<210>  18
<211>  65
<212>  DNA
<213>  chloroplast

<400>  18
ggagaagaag caatgacggt atctggggaa taagcatcgg ctaactctgt gccagcagcc      60

gcggt                                                                 65


<210>  19
<211>  65
<212>  DNA
<213>  chloroplast

<400>  19
ggagaagaag caatgacggt atctggggaa taagcatcgg ctaactctgt gccagcagcc      60

gcggt                                                                 65


<210>  20
<211>  65
<212>  DNA
<213>  chloroplast

<400>  20
ggagaagaag caatgacggt atctggggaa taagcatcgg ctaactctgt gccagcagcc      60

gcggt                                                                 65


<210>  21
<211>  65
<212>  DNA
<213>  chloroplast

<400>  21
ggagaagaag caatgacggt atctggggaa taagcatcgg ctaactctgt gccagcagcc      60

gcggt                                                                 65


<210>  22
<211>  65
<212>  DNA
<213>  chloroplast

<400>  22
ggagaagaag caatgacggt atctggggaa taagcatcgg ctaactctgt gccagcagcc      60

gcggt                                                                 65


<210>  23
<211>  65
<212>  DNA

<213>  chloroplast

<400>  23
agagaagaag caatgacggt atctggggaa taagcatcgg ctaactctgt gccagcagcc      60

gcggt                                                                  65


<210>  24
<211>  65
<212>  DNA
<213>  chloroplast

<400>  24
ggagaagaaa caatgacggt atctgaggaa taagcatcgg ctaactctgt gccagcagcc      60

gcggt                                                                  65


<210>  25
<211>  65
<212>  DNA
<213>  chloroplast

<400>  25
ggagaagaaa caatgacggt atctgaggaa taagcatcgg ctaactccgt gccagcagcc      60

gcggt                                                                  65


<210>  26
<211>  88
<212>  DNA
<213>  Pseudomonas

<400>  26
tgggaggaag ggcagtaagc gaataccttg ctgttttgac gttaccgaca gaataagcac      60

cggctaactc tgtgccagca gccgcggt                                         88


<210>  27
<211>  88
<212>  DNA
<213>  Xanthomonas

<400>  27
ggggaagaaa tcctgctggc taatatccgg cggggatgac ggtacccaaa gaataagcac      60

cggctaactt cgtgccagca gccgcggt                                         88


<210>  28
<211>  88
<212>  DNA
<213>  Escherichia coli

<400>  28
ggggaggaag ggagtaaagt taataccttt gctcattgac gttacccgca gaagaagcac      60

cggctaactc cgtgccagca gccgcggt                                         88

<210>  29
<211>  88
<212>  DNA
<213>  Lactococcus

<400>  29
agagaagaaa gtgcgtgaga gtaactgttc acgtttcgac ggtatctaac cagaaagcca      60

cggctaacta cgtgccagca gccgcggt                                        88


<210>  30
<211>  88
<212>  DNA
<213>  Brochothrix


<220>
<221>  misc_feature
<222>  (34)..(34)
<223>  n is a, c, g, or t

<400>  30
agagaagaac atgggtgaga gtaactgttc accncttgac ggtatctaac cagaaagcca      60

cggctaacta cgtgccagca gccgcggt                                        88


<210>  31
<211>  79
<212>  DNA
<213>  Arthrobacter

<400>  31
agggtagaag cctgtcttag gggtcggtga cggtacctgc agaagaagcg ccggctaact      60

acgtgccagc agccgcggt                                                  79


<210>  32
<211>  17
<212>  DNA
<213>  consensus

<400>  32
arcaatgacg gtatctg                                                    17


<210>  33
<211>  45
<212>  DNA
<213>  Oryza sativa

<400>  33
ctgtttgatg agcctgcgta gtattaggta gttggtcggg taaag                     45


<210>  34
<211>  45
<212>  DNA
<213>  Oryza rufipogon

<400>  34

ctgtttgatg agcctgcgta gtattaggta gttggtcggg taaag 45

<210> 35
<211> 45
<212> DNA
<213> Bambusa oldhamii

<400> 35
ctgtttgatg agcctgcgta gtattaggta gttggtcagg taaag 45

<210> 36
<211> 45
<212> DNA
<213> Triticum aestivum

<400> 36
ctgtttgatg agcctgcgta gtattaggta gttggtcagg taaag 45

<210> 37
<211> 45
<212> DNA
<213> S. cereale

<400> 37
ctgtttgatg agcctgcgta gtattaggta gttggtcagg taaag 45

<210> 38
<211> 45
<212> DNA
<213> Sorghum bicolor

<400> 38
ctgtttgatg agcctgcgta gtattaggta gttggtcagg taaag 45

<210> 39
<211> 46
<212> DNA
<213> chloroplast

<400> 39
gcccgaggag gggctcgcgt ctgattagct agttggtgag gcaata 46

<210> 40
<211> 46
<212> DNA
<213> chloroplast

<400> 40
gcccgaggag gggctcgcgt ctgattagct agttggtgag gcaata 46

<210> 41
<211> 46
<212> DNA
<213> chloroplast

<400> 41

gcccaaggag gggctcgcgt ctgattagct agttggtgag gcaata                    46

<210>  42
<211>  46
<212>  DNA
<213>  chloroplast

<400>  42
gcccaaggag gggctcgcgt ctgattagct agttggtgag gcaata                    46

<210>  43
<211>  47
<212>  DNA
<213>  Escherichia coli

<400>  43
tgccatcgga tgtgcccaga tgggattagc ttgttggtgg ggtaacg                   47

<210>  44
<211>  50
<212>  DNA
<213>  Lactococcus lactis

<400>  44
tatcactttg ggatggacct gcggcgcatt agcttgttgg tagggtaacg                50

<210>  45
<211>  50
<212>  DNA
<213>  weissella

<400>  45
tatcactaag agatggtccc gcggtgcatt agctagttgg taaggtaatg                50

<210>  46
<211>  50
<212>  DNA
<213>  Brochothrix thermosphacta

<400>  46
tgtcactgtg agatggaccc gcgctggatt agctagttgg taaggtaatg                50

<210>  47
<211>  50
<212>  DNA
<213>  staphylococcus sp

<400>  47
tatcacttat agatggaccc gcgccgtatt agctagttgg taaggtaacg                50

<210>  48
<211>  18
<212>  DNA
<213>  consensus

<400>  48

tgtttgatga gcctgcgt                                                    18

<210>  49
<211>  49
<212>  DNA
<213>  Lactobacillus delbrueckii

<400>  49
ggcctaccaa ggcaatgatg cgtagccgag ttgagagact gatcggcca                 49

<210>  50
<211>  49
<212>  DNA
<213>  Lactobacillus delbrueckii

<400>  50
ggcctaccaa ggcaatgatg cgtagccgag ttgagagact gatcggcca                 49

<210>  51
<211>  49
<212>  DNA
<213>  Lactobacillus amylovorus

<400>  51
ggcttaccaa ggcgacgatg catagccgag ttgagagact gatcggcca                 49

<210>  52
<211>  49
<212>  DNA
<213>  Lactobacillus acidophilus

<400>  52
ggcctaccaa ggcaatgatg catagccgag ttgagagact gatcggcca                 49

<210>  53
<211>  49
<212>  DNA
<213>  Lactobacillus amyloticus

<400>  53
ggcttaccaa ggcaacgatg catagccgag ttgagagact gatcggcca                 49

<210>  54
<211>  49
<212>  DNA
<213>  Lactobacillus acetotolerans

<400>  54
ggcttaccaa ggcaacgatg catagccgag ttgagagact gatcggcca                 49

<210>  55
<211>  49
<212>  DNA
<213>  Lactococcus piscium

<400>  55

ggactaccaa ggcgatgata catagccgac ctgagagggt gatcggcca 49

<210> 56
<211> 49
<212> DNA
<213> Lactococcus plantarum

<400> 56
ggactaccaa ggcgatgata catagccgac ctgagagggt gatcggcca 49

<210> 57
<211> 49
<212> DNA
<213> Lactococcus chungangensis

<400> 57
ggactaccaa ggcgatgata catagccgac ctgagagggt gatcggcca 49

<210> 58
<211> 49
<212> DNA
<213> Lactococcus raffinolactis

<400> 58
ggactaccaa ggcgatgata catagccgac ctgagagggt gatcggcca 49

<210> 59
<211> 49
<212> DNA
<213> Lactococcus-lactis-subsp-Hordn

<400> 59
ggctcaccaa ggcgatgata catagccgac ctgagagggt gatcggcca 49

<210> 60
<211> 49
<212> DNA
<213> Lactococcus-lactis-subsp-Lacti

<400> 60
ggctcaccaa ggcgatgata catagccgac ctgagagggt gatcggcca 49

<210> 61
<211> 49
<212> DNA
<213> Lactococcus-lactis-subsp-Cremo

<400> 61
ggctcaccaa ggcgatgata catagccgac ctgagagggt gatcggcca 49

<210> 62
<211> 49
<212> DNA
<213> Streptococcus thermophilus

<400> 62

ggctcaccaa ggcgacgata catagccgac ctgagagggt gatcggcca 49

<210> 63
<211> 49
<212> DNA
<213> Streptococcus thermophilus

<400> 63
ggctcaccaa ggcgatgata catagccgac ttgagagggt gatcggcca 49

<210> 64
<211> 49
<212> DNA
<213> Streptococcus thermophilus

<400> 64
ggctcaccta ggcgacgata catagccgac ctgagagggt gatcggcca 49

<210> 65
<211> 49
<212> DNA
<213> Streptococcus parauberis

<400> 65
ggctcaccaa ggccacgata catagccgac ctgagagggt gatcggcca 49

<210> 66
<211> 49
<212> DNA
<213> Streptococcus iniae

<400> 66
ggctcaccaa ggcgacgata catagccgac ctgagagggt gatcggcca 49

<210> 67
<211> 49
<212> DNA
<213> Streptococcus dysgalactiae

<400> 67
ggctcaccaa ggcgacgata catagccgac ctgagagggt gatcggcca 49

<210> 68
<211> 49
<212> DNA
<213> Streptococcus agalactiae

<400> 68
ggctcaccaa ggcgacgata catagccgac ctgagagggt gatcggcca 49

<210> 69
<211> 49
<212> DNA
<213> Streptococcus equi

<400> 69

ggcctaccaa ggcgacgata catagccgac ctgagagggt gaacggcca 49

<210> 70
<211> 49
<212> DNA
<213> Streptococcus oligofermentans

<400> 70
ggctcaccta ggcgacgata catagccgac ctgagagggt gatcggcca 49

<210> 71
<211> 49
<212> DNA
<213> Enterococcus faecalis

<220>
<221> misc_feature
<222> (11)..(11)
<223> n is a, c, g, or t

<220>
<221> misc_feature
<222> (13)..(13)
<223> n is a, c, g, or t

<400> 71
ggctcaccaa ngncacgatg catagccgac ctgagagggt gatcggcca 49

<210> 72
<211> 49
<212> DNA
<213> Lactobacillus algidus

<400> 72
ggcccaccaa ggcaatgata cgtagccgac ctgagagggt gatcggcca 49

<210> 73
<211> 49
<212> DNA
<213> Lactobacillus frumenti

<400> 73
ggcttaccaa ggcgatgatg catagccgag ttgagagact gatcggcca 49

<210> 74
<211> 49
<212> DNA
<213> Lactobacillus panis

<400> 74
ggcttaccaa ggcaatgatg catagccgag ttgagagact gatcggcca 49

<210> 75
<211> 49
<212> DNA
<213> Lactobacillus fermentum

<400>  75
ggcctaccaa ggcgatgatg catagccgag ttgagagact gatcggcca                    49


<210>  76
<211>  49
<212>  DNA
<213>  Lactobacillus oligofermentans

<400>  76
ggctcaccaa gacaatgata cgtagccgaa ctgagaggtt gatcggcca                    49


<210>  77
<211>  49
<212>  DNA
<213>  Lactobacillus plantarum

<400>  77
ggctcaccat ggcaatgata cgtagccgac ctgagagggt aatcggcca                    49


<210>  78
<211>  49
<212>  DNA
<213>  Brevibacterium linens

<400>  78
ggcctaccaa ggcgacgacg ggtagccggc ctgagagggc gaccggcca                    49


<210>  79
<211>  49
<212>  DNA
<213>  Psychroflexus sp

<400>  79
ggcttaccat ggcaacgata ggtaggggtc ctgagaggga gatcccccca                    49


<210>  80
<211>  18
<212>  DNA
<213>  consensus

<400>  80
gatgatacat agccgacc                                                      18


<210>  81
<211>  1501
<212>  DNA
<213>  Escherichia coli

<400>  81
gagagtttga tcatggctca gattgaacgc tggcggcagg cctaacacat gcaagtcgaa        60

cggtaacagg aagcagcttg cttctttgct gacgagtggc ggacgggtga gtaatgtctg       120

ggaaactgcc tgatggaggg ggataactac tggaaacggt agctaatacc gcataacgtc       180

gcaagaccaa agagggggac cttcgggcct cttgccatcg gatgtgccca gatgggatta       240

gctagtaggt ggggtaacgg ctcacctagg cgacgatccc tagctggtct gagaggatga 300

ccagccacac tggaactgag acacggtcca gactcctacg ggaggcagca gtggggaata 360

ttgcacaatg ggcgcaagcc tgatgcagcc atgccgcgtg tatgaagaag gccttcgggt 420

tgtaaagtac tttcagcggg gaggaaggga gtaaagttaa tacctttgct cattgacgtt 480

acccgcagaa gaagcaccgg ctaactccgt gccagcagcc gcggtaatac ggagggtgca 540

agcgttaatc ggaattactg ggcgtaaagc gcacgcagcg gtttgttaag tcagatgtga 600

aatccccggg ctcaacctgg gaactgcatc tgatactggc aagcttgagt ctcgtagagg 660

ggggtagaat tccaggtgta gcggtgaaat gcgtagagat ctggaggaat accggtggcg 720

aaggcggccc cctggacgaa gactgacgct caggtgcgaa agcgtgggga gcaaacagga 780

ttagataccc tggtagtcca cgccgtaaac gatgtcgact tggaggttgt gcccttgagg 840

cgtggcttcc ggagctaacg cgttaagtcg accgcctggg gagtacggcc gcaaggttaa 900

aactcaaatg aattgacggg ggcccgcaca agcggtggag catgtggttt aattcgatgc 960

aacgcgaaga accttacctg gtcttgacat ccacggaagt tttcagagat gagaatgtgc 1020

cttcgggaac cgtgagacag gtgctgcatg gctgtcgtca gctcgtgttg tgaaatgttg 1080

ggttaagtcc cgcaacgagc gcaaccctta tcctttgttg ccagcggtcc ggccgggaac 1140

tcaaaggaga ctgccagtga taaactggag gaaggtgggg atgacgtcaa gtcatcatgg 1200

cccttacgac cagggctaca cacgtgctac aatggcgcat acaaagagaa gcgacctcgc 1260

gagagcaagc ggacctcata aagtgcgtcg tagtccggat tggagtctgc aactcgactc 1320

catgaagtcg gaatcgctag taatcgtgga tcagaatgcc acggtgaata cgttcccggg 1380

ccttgtacac accgcccgtc acaccatggg agtgggttgc aaaagaagta ggtagcttaa 1440

ccttcgggag ggcgcttacc actttgtgat tcatgactgg ggtgaagtcg taacaaggta 1500

a 1501

<210> 82
<211> 51
<212> DNA
<213> consensus

<400> 82
cctatcccct gtgtgccttg gcagtctcag gagagtttga tymtggctca g 51

<210> 83
<211> 58
<212> DNA
<213> consensus

<220>
<221> misc_feature

<222>  (31)..(40)
<223>  n is a, c, g, or t

<400>  83
ccatctcatc cctgcgtgtc tccgactcag nnnnnnnnnn taccgcggct gctggcac          58

## Description

The invention relates to an analytical method that has been developed to identify and quantify the total microorganism content of any kind of samples including *inter alia* food samples and stool samples.

Microbial contents of food, faeces or environmental samples are frequently either identified or quantified through culture dependant methods. Among new generation molecular technologies the metagenomic analysis has emerged as a powerful tool. Metagenomics applies a suite of genomic technologies and bioinformatics tools to directly access the genetic content of entire communities of organisms. This technology is commonly used to describe microbial community profiles of various ecosystems like seas, soils, rumen, and faeces but also to study complex microbiota of foodstuff.

Several problems remain with metagenomic analysis of foodstuffs and other like samples. Firstly, it is necessary to provide a global metagenomic approach which is not dependant on the microbe's ability to grow on determined culture media at any define environmental conditions (e.g. pH, T°, Aw). Such an approach should provide for the description as well as quantification of the observed microorganisms, which is of critical needs in some environments, for example when food spoilage issues are to be explained.

Secondly, prior art methods of metagenomic analysis may be biased by the presence in the samples being tested of non-viable microorganisms. In many cases, particularly in the metagenomic analysis of food samples, it is important to be able to characterise and quantify only the live microorganisms in a sample. Prior art metagenomic techniques do not allow for such an analysis and results, when applied to food sample analysis may therefore be misleading.

Thirdly, prior art methods of metagenomic analysis may be biased by the presence of contaminating DNA present in the sample, which is amplified together with the target sequence of interest, but which does not provide useful information on the microbiota of the sample. For example chloroplast DNA or mitochondrial DNA may co-amplify with the target, where the target is the well known 16S rRNA gene.

Fourthly, prior art methods of metagenomic analysis may be biased by the presence of a large amount of DNA from bacteria or other microorganisms deliberately added to foodstuff as an ingredient or by the presence of a natural dominant species. Such DNA can mask the DNA of interest in a metagenomic analysis.

There is therefore a need for improved metagenomic analysis methods and tools. The invention overcomes these and other problems.

### Summary of the Invention

The invention therefore provides an integrated metagenomic approach developed to (I) identify and (II) quantify the micro-organism content of any samples, (III) distinguish if the observed DNA sequences derived from dead or alive germs, (IV) block the well known co-amplification of mitochondrial and plastid DNA with universal bacterial 16S primers, that might represent more than 90 % of the sequences in some food preparations, and (V) block the amplification of over-represented germs avoiding rare but important germs to be observed.

Our metagenomic analysis is a PCR-based approach targeting the highly conserved rDNA locus encoding for the 16S rDNA gene. This gene is commonly used for bacterial taxonomic classification as it contains a succession of nine hyper variable regions with well-conserved sequences in between. The conserved sequences are used to design universal bacterial primers flanking a polymorphic region chosen based on its high information content for a precise species identification of microorganism content.

In a first aspect, the invention provides a method of metagenomic analysis of a food or stool sample to detect and quantitate microorganisms present in said sample, the analysis comprising amplification of the V2-V3 region of bacterial 16S rRNA in said sample. In some embodiments the V1-V3 region of bacterial 16S rRNA is amplified. The inventors have identified that the V2-V3 region provides excellent hypervariability between common food spoilage flora, and therefore allows for bacteria likely to be present in food samples to be distinguished and quantitated.

In some embodiments, the use of V2-V3 of 16S rRNA, according to the invention, in a metagenomic analysis, allows for the identification and distinguishing of sequences from bacteria of the families: *Acetobacteraceae, Aeromonadaceae, Bacteroidaceae, Burkholderiaceae, Carnobacteriaceae, Clostridiaceae, Comamonadaceae, Enterobacteriaceae, Enterococcaceae, Flavobacteriaceae, Lachnospiraceae, Lactobaillaceae, Leuconostocaceae, Listeriaceae, Moraxellaceae, Neisseriaceae, Pseudoalteromonadaceae, Pseudomonadaceae, Shewanellaceae, Streptococcaceae, Vibrionaceae* and *Xanthomonadaecea.*

In more detail, the invention allows for the detection and distinguishing of bacteria of the genera: *Acetobacteraceae Acetobacter, Acetobacteraceae Gluconacetobacter, Aeromonadaceae Aeromonas, Bacteroidaceae Bacteroides, Burkholderiaceae Burkholderia, Carnobacteriaceae Carnobacterium, Clostridiaceae Clostridium, Comamonadaceae Comamonas, Comamonadaceae Delftia, Enterobacteriaceae Escherichia, Enterobacteriaceae Pantoea, Enterobacteriaceae Serratia, Enterococcaceae Enterococcus, Chryseobacterium, Flavobacteriaceae Flavobacterium, Flavobacteriaceae Myroides, Lachnospiraceae Butyrivibrio, Lactobaillaceae Lactobacillus, Lactobacillaceae Pediococcus, Leuconostocaceae Leuconostoc, Leuconostocaceae Weissella, Listeriaceae Listeria, Moraxellaceae Acinetobacter, Moraxellaceae Psychrobacter, Neisseriaceae Alysiella, Pseudoalteromonadaceae Pesudoalteromonas, Pseudomonadaceae Pseudomonas, Shewanellaceae Shewanella, Streptococcaceae Lactococcus, Streptococcaceae Streptococcus, Vibrionaceae Aliivibrio, Vibrionaceae Photobacterium, Vibrionaceae Vibrio* and *Xanthomonadaecea Xanthomonas.*

Metagenomic analysis of the DNA present in a sample may provide a relative quantitative analysis of different microorganisms present. However in preferred embodiments the amount of some, or each of the microorganisms detected is normalised and expressed as a percentage of the colony forming units in the sample. This may be important in some embodiments since the absolute relative amounts of microorganisms in a sample may not be indicative of the spoilage potential of the sample or the shelf life limit. The invention therefore allows one to accurately qualify the quality of a food sample.

Normalisation of the quantity of microorganisms in a sample may be carried out by determining the percentage of microorganisms represented by at least one operational taxonomic unit (OTU) in the sample. This may be combined with determining the colony forming units count (CFU) of the sample and normalising the percentage of organisms represented by said OTU to a proportion of the total viable CFU count of the sample.

Further quantitation of DNA in the sample may be provided, in some embodiments, by conducting taxa specific quantitative PCR (qPCR) on samples following metagenomic analysis. In such embodiments, the taxa chosen for the qPCR analysis are those taxa of interest identified by the metagenomic analysis. Any form of qPCR may be employed using any suitable gene, and any detection and quantification process. For example the 16S rRNA gene may be amplified in qPCR tests. Different qPCR tests that might be employed include those involving an intercalating dye, and those involving a labelled reporter primer or probe. For example qPCR may be conducted using a hydrolysis probe (e.g. a Taqman probe), a molecular beacon probe, a pair of dual hybridisation probes, an amplifluour primer system, a scorpion primer system, a light-upon-extension system or a QZyme primer system.

In some embodiments the V2-V3 region of 16S rRNA is amplified in the metagenomic analysis as part of a larger fragment of the 16S rRNA gene. For example, in some embodiments, the V1-V3 region is amplified.

In some embodiments one or both primers having sequences comprising the sequences of SEQ ID NOs 1 and/or 2 may be used to amplify the V1-V3 region. Preferably, a pair of primers comprising the sequences of SEQ ID NO. 1 and SEQ ID NO. 2 respectively are used to amplify the V1-V3 region. In some embodiments the primers consist of the sequences of SEQ ID NOs 1 and 2 respectively.

Such primers may be modified by the addition of adaptors, key sequences, tags or universal sequences. In some embodiments, at least one of the primers has the sequence of SEQ ID NO. 82. In some embodiments, at least one of the primers as the sequence of SEQ ID NO. 83. In some embodiments a pair of primers having the sequences of SEQ ID NO.s 82 and 83 respectively is used.

In order to improve the analytical power of metagenomic analysis of a sample, the invention provides methods in which the amplification of plastid or mitochondrial DNA, and/or DNA from selected microorganisms is reduced. The invention therefore provides, in a further aspect, the use of Peptide Nucleic Acid (PNA) clamping in a method of metagenomic analysis of a sample, to reduce amplification of at least one of: i) chloroplast DNA present in the sample; ii) mitochondrial DNA present in the sample; or iii) DNA from one or more selected microorganisms that may be present in the sample.

In some embodiments, the PNA clamping comprises conducting metagenomic analysis in the presence of a PNA oligonucleotide comprises a sequence derived from a chloroplast 16S rRNA gene, preferably a sequence common to a plurality of chloroplast 16S rRNA genes.

In some embodiments the PNA oligonucleotide comprises a sequence common to at least two of, and preferably all of, the chloroplast 16S rRNA sequences of SEQ ID NOs 3 to 10, or common to at least two of, and preferably all of, the chloroplast 16S rRNA sequences of SEQ ID NOs 18 to 25.

In some embodiments, the PNA olignucleotide has a sequence not found in the bacterial 16S rRNA sequences of at least one of, and preferably all of, SEQ ID NOs 11 to 16, or has a sequence not found in the bacterial 16S rRNA sequences of at least one of, and preferably all of, SEQ ID NOs 26 to 31.

In some embodiments, the PNA oligonucleotide comprises the sequence of SEQ ID NO. 17. Preferably the PNA oligonucleotide consists of the sequence of SEQ ID NO. 17.

In some embodiments, the PNA oligonucleotide comprises the sequence of SEQ ID NO. 32. Preferably, the PNA oligonucleotide consists of the sequence of SEQ ID NO. 32.

In some embodiments the metagenomic analysis is conducted in the presence of a pair of PNA oligonucleotides comprising of or consisting of the sequences of SEQ ID NO.s 17 and 32 respectively.

In some embodiments, PNA clamping comprises conducting metagenomic analysis in the presence of a PNA oligonucleotide comprises a sequence derived from a mitochondrial 18S rRNA gene, preferably a sequence common to a plurality of mitochondrial 18S rRNA genes.

In some embodiments, the PNA oligonucleotide comprises a sequence common to at least two of, and preferably all of, the mitochondrial 18S rRNA sequences of SEQ ID NOs 33 to 38.

In some embodiments, PNA olignucleotide has a sequence not found in the chloroplast 16S rRNA sequences, or the bacterial 16S rRNA sequences of at least one of, and preferably all of, SEQ ID NOs 39 to 47.

In some embodiments, the PNA oligonucleotide comprises the sequence of SEQ ID NO. 48, optionally wherein said PNA oligonucleotide consists of the sequence of SEQ ID NO. 48.

In some embodiments, the PNA clamping comprises conducting metagenomic analysis in the presence of a PNA oligonucleotide comprises a sequence derived from a bacterial 16S rRNA gene, preferably a sequence common to a plurality of bacterial 16S rRNA genes.

In some embodiments, the PNA oligonucleotide comprises a sequence common to at least two of, and preferably all of, the bacterial 16S rRNA sequences of SEQ ID NOs 49 to 61.

In some embodiments, the PNA olignucleotide has a sequence not found in the bacterial 16S rRNA sequences of at least one of, and preferably all of, SEQ ID NOs 62 to 79.

In some embodiments, the PNA oligonucleotide comprises the sequence of SEQ ID NO. 80, optionally wherein said PNA oligonucleotide consists of the sequence of SEQ ID NO. 80.

In some embodiments the metagenomic analysis is conducted in the presence of any two, three or four of the PNA oligonucleotides discussed above, that is any two, three or four PNA oligonucleotides comprising or consisting of the sequences of SEQ ID NO.s 17, 32, 48 or 80 respectively.

The invention is further directed towards the ability to distinguish the amplification of DNA from live and dead microorganisms in a metagenomic analysis of a sample. The invention achieves this by the use of the intercalating dye propidium monoazide which penetrates dead cells in the sample prior to DNA extraction, intercalates with the DNA of those dead cells and prevents subsequent amplification. The invention therefore provides, in a further aspect, a method of metagenomic analysis of a sample, wherein propidium monoazide (PMA) is added to the sample prior to DNA extraction in order to reduce the subsequent amplification of DNA from non-viable microorganisms present in the sample.

In each an every one of the uses of PNA clamping described herein or the use of PMA as described herein, the metagenomic analysis may be carried out in accordance with any other embodiment or aspect of the invention described herein.

In a yet further aspect, the invention provides a kit for metagenomic analysis of a sample comprising one or both primers comprising the sequence of SEQ ID NO. 1 and/or SEQ ID NO. 2, preferably wherein the primers consist of the sequences of SEQ ID NO. 1 and/or SEQ ID NO. 2.

In some embodiments, the primers of the kit may be modified by the addition of an adaptor, key sequence, tag or universal sequence. Preferably, the primers have the sequences of SEQ ID NO.s 82 and 83 respectively.

In some embodiments, a kit of the invention further comprises a PNA oligonucleotide comprising a sequence of any of SEQ ID NOs. 17, 32, 48 or 80, preferably wherein the primer consists of the sequence of any of SEQ ID NOs. 17, 32, 48 or 80. Preferably the kit may comprise a set of primers including any two, three or four PNA oligonucleotides comprising or consisting of the sequence of SEQ ID NO.s 17, 32, 48 or 80.

In some embodiments the kit of the invention may further comprise PMA.

In some embodiments the kit of the invention may further comprise a reagent for metagenomic analysis of a sample, e.g. a food or stool sample.

The invention will now be described in more detail with reference to the following illustrative Examples and the figures, in which:
Figure 1 shows the nine hypervariable regions of the 16S rRNA gene.
Figure 2 shows taxonomic information content along the whole 16S gene.
Figure 3 shows the sequence of *Escherichia coli* 16S ribosomal RNA gene (Genebank Accession Number AY804014.1, SEQ ID NO. 81) with annotation of the polymorphism frequencies across the bacterial kingdom.
Figure 4 shows primer statistics of both primers and amplicon product size, and the sequences of the forward and reverse primers (SEQ ID NO. 1 and SEQ ID NO. 2 respectively).

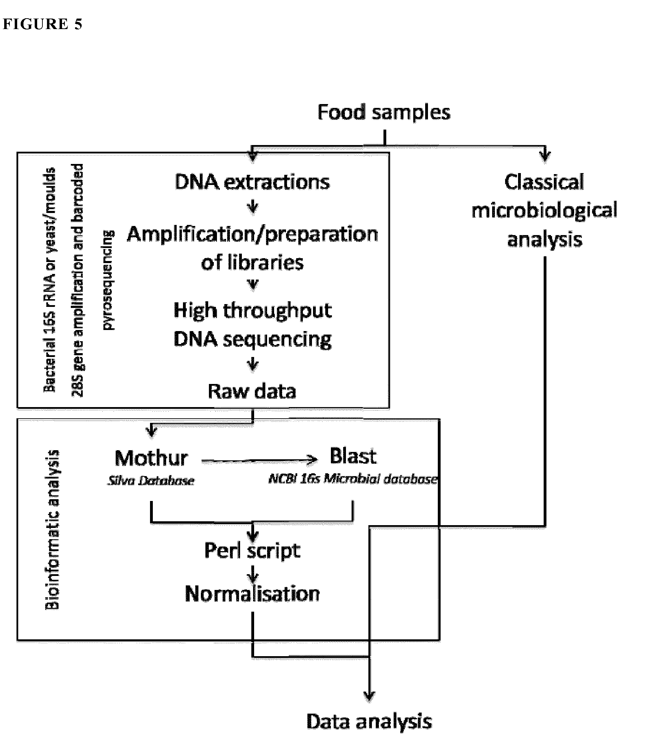

Figure 5 shows a flow diagram of the steps involved in the metagenomic analysis
Figure 6 shows an alignment of chloroplast 16S RNA sequences with various bacterial 16S rRNA sequences and the sequence of a first PNA clamp. Sequences entitled Chloroplast 1 to Chloroplast 8 are SEQ ID NO.s 3 to 10 respectively. SEQ ID NO.s of the other sequences are: *Pseudomonas* SEQ ID NO. 11; *Xanthomonas* SEQ ID NO. 12; *E*. *coli* SEQ ID NO. 13; *Lactococcus* SEQ ID NO. 14; *Brochothrix* SEQ ID NO. 15; *Artrhrobacter* SEQ ID NO 16. the PNA oligonucleotide QP-PNAcup is SEQ ID NO. 17.
Figure 7 shows a further alignment of chloroplast 16S RNA sequences with various bacterial 16S rRNA sequences and the sequence of a second PNA clamp. Sequences entitled Chloroplast 1 to Chloroplast 8 are SEQ ID NO.s 18 to 25 respectively. SEQ ID NO.s of the other sequences are: *Pseudomonas* SEQ ID NO. 26; *Xanthomonas* SEQ ID NO. 27; *E. coli* SEQ ID NO. 28; *Lactococcus* SEQ ID NO. 29; *Brochothrix* SEQ ID NO. 30; *Artrhrobacter* SEQ ID NO 31. the PNA oligonucleotide QP-PNAcdn is SEQ ID NO. 32.

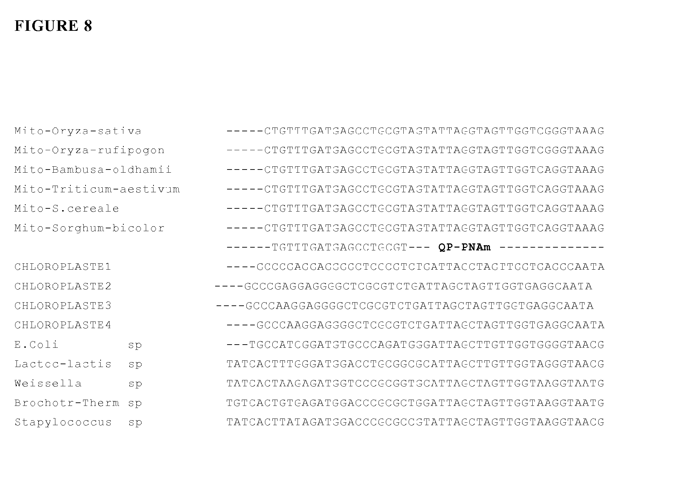

Figure 8 shows an alignment of mitochondrial 18S RNA sequences with various bacterial 16S rRNA sequences and the sequence of a third PNA clamp. SEQ ID NOs. of the sequences shows are: *Mito-Oryza-sativa* SEQ ID NO. 33; *Mito-Oryza-rufipogon* SEQ ID NO. 34; *Mito-Bambusa-oldhamii* SEQ ID NO. 35; *Mito-Triticum-aestivum* SEQ ID NO. 36; *Mito-S.cereale* SEQ ID NO.37; *Mito-Sorghum-bicolor* SEQ ID NO. 38; Chloroplast E1 to Chloroplast E4 are SEQ ID NOs. 39 to 42 respectively; *E. coli* SEQ ID NO. 43; *Lactococcus lactis* SEQ ID NO. 44; *Weissella* SEQ ID NO. 45; *Brochotrix thermosphacta* SEQ ID NO. 46; *Staphylococcus* SEQ ID NO.47. The PNA oligonucleotide QP-PNAm is SEQ ID NO. 48.

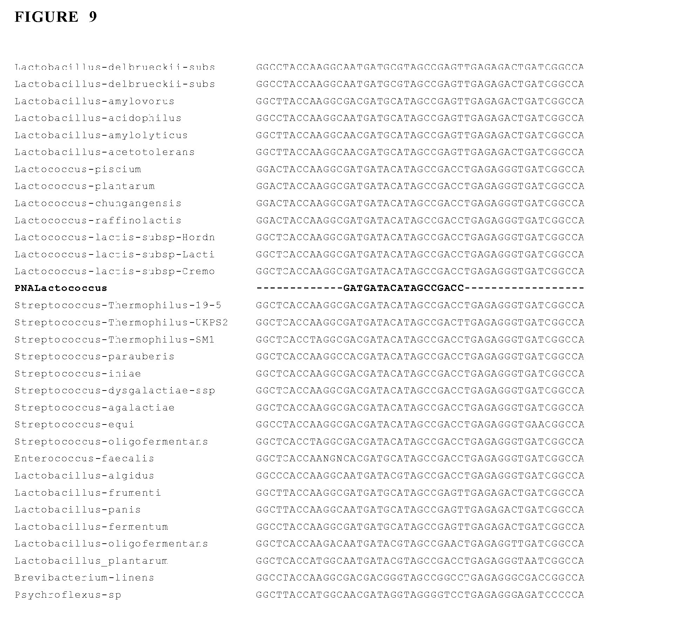

Figure 9 shows an alignment of 16S rRNA sequences from various bacteria of the lactococcus taxa, together with various contaminating bacteria, and a fourth PNA clamp. The SEQ ID NOs. of the sequences shown are: *Lactobacillus-delbrueckii-subs* SEQ ID NO.49; *Lactobacillus-delbrueckii-subs* SEQ ID NO. 50; *Lactobacillus-amylovorus* SEQ ID NO. 51; *Lactobacillus-acidophilus* SEQ ID NO. 52; *Lactobacillus-amylolyticus* SEQ ID NO. 53; *Lactobacillus-acetotolerans* SEQ ID NO. 54; *Lactococcus-piscium* SEQ ID NO. 55; *Lactococcus-plantarum* SEQ ID NO. 56; *Lactococcus-chungangensis* SEQ ID NO. 57; *Lactococcus-raffinolactis* SEQ ID NO. 58; *Lactococcus-lactis-subsp-Hordn* SEQ ID NO. 59; *Lactococcus-lactis-subsp-Lacti* SEQ ID NO. 60; *Lactococcus-lactis-subspCremo* SEQ ID NO. 61; *Streptococcus-Thermophilus-19-5* SEQ ID NO. 62; *Streptococcus-Thermophilus-UKPS2* SEQ ID NO. 63; *Streptococcus-Thermophilus-SM1* SEQ ID NO. 64; *Streptococcus-parauberis* SEQ ID NO. 65; *Streptococcus-iniae* SEQ ID NO. 66; *Streptococcus-dysgalactiae-ssp* SEQ ID NO. 67; *Streptococcus-agalactiae* SEQ ID NO. 68; *Streptococcus-equi* SEQ ID NO. 69; *Streptococcus-oligofermentans* SEQ ID NO. 70; *Enterococcus-faecalis* SEQ ID NO. 71; *Lactobacillus-algidus* SEQ ID NO.72; *Lactobacillus-frumenti* SEQ ID NO. 73; *Lactobacillus-panis* SEQ ID NO.74; *Lactobacillus-fermentum* SEQ ID NO. 75; *Lactobacillus-oligofermentans* SEQ ID NO. *76; Lactobacillus_plantarum* SEQ ID NO. 77; *Brevibacterium-linens* SEQ ID NO. 78 *Psychroflexus-sp* SEQ ID NO. 79. The PNA oligonucleotide PNALactococcus is SEQ ID NO. 80.
Figure 10 shows metagenomic analysis of steak tartare samples.
Figure 11 shows relative proportions of the major bacterial taxa (over 1% of prevalence) in steak tartare samples.
Figure 12 shows a representation of the converted relative bacterial count of the OTUs in total count numbers of population. Only taxa whose population reached 10,000 cells per gram are visible. The bold bar indicates the threshold of 6 logs of CFUs above which the spoilage activity would be noticeable.
Figure 13 shows a comparison between bacterial content of 3 probiotics with and without PMA treatment.
Figure 14 shows an analysis of a pasta product with the standard metagenomic protocol, with the 2 PNA clamps specific to avoid plastid DNA, with the PNA directed against the mitochondrial DNA and finally with the 3 PNA clamps together.
Figure 15 shows bacterial profiles of 2 cheeses obtained with and without the use of a PNALactococcus clamping probe.

### Examples

### Material and methods

### DNA isolation

Twenty-five gram sample of meat product are homogenized for 1 minute in 225 ml in a tempo bag (bioMérieux Basingstoke, England, ref 80015) with sterile physiologic water using a stomacher apparatus. 1.5 ml of the suspension are then used for total genomic DNA extraction using a home made lysis step prior to the conventional use of the DNeasy Blood & Tissue Kit (QIAGEN, Crawley, UK). The DNA concentration is then evaluated by fluorometric dsDNA quantification using PicoGreen.

### Descriptive metagenomic analysis

### - Amplicon selection and primer designs

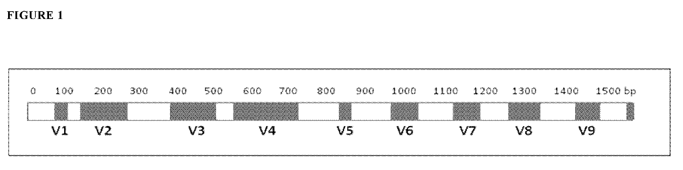

Culture-independent 16S rRNA gene sequencing has been widely applied to examine microbial diversity. Bacterial 16S ribosomal RNA (rRNA) genes contain nine "hypervariable regions" (V1-V9) (Figure 1), that demonstrate considerable sequence diversity among different bacteria. Unfortunately, 16S rRNA hypervariable regions exhibit different degrees of sequence diversity, and no single hypervariable region is able to distinguish among all bacteria. The full-length 16S rRNA genes (about 1500 bp) can be used for accurate taxonomic identification based on the underlying sequence diversity among different bacterial species. However, the current high-throughput DNA sequencing technologies can only produce 16S rRNA gene fragments, instead of full-length genes. The challenge is to obtain reliable representation of bacterial communities through taxonomic classification of short 16S rRNA gene sequences

The inventors have discovered the most appropriate region for food metagenomic analysis. Information content of 16S rRNA, for bacterial assignation to the species level, were analyzed for different 16S regions across the most frequent food taxa.

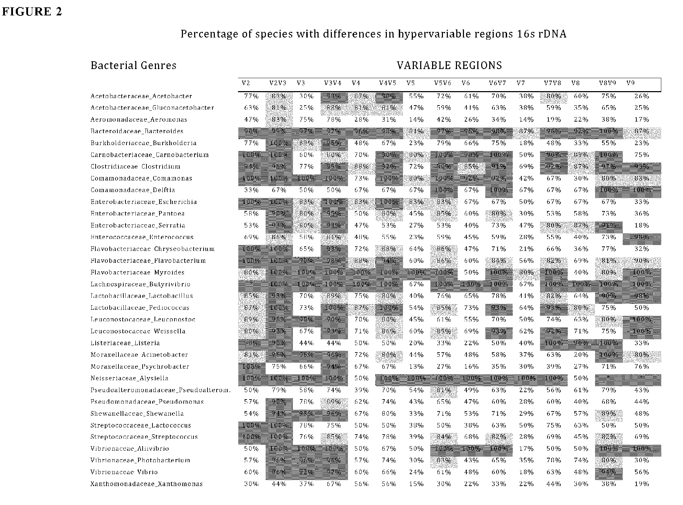

For this purpose, reference sequences from the most common food bacterial taxa were extracted from open databases (Silva) and in silico cut in various fragment containing the sequences of a single or two contagious hypervariable regions. After comparing those fragments to the databases, percentages of taxonomical identification to the species were computed for each region. As can be seen in figure 2, the V2-V3 region seems to be the more informative with respect to the common food spoilage flora.

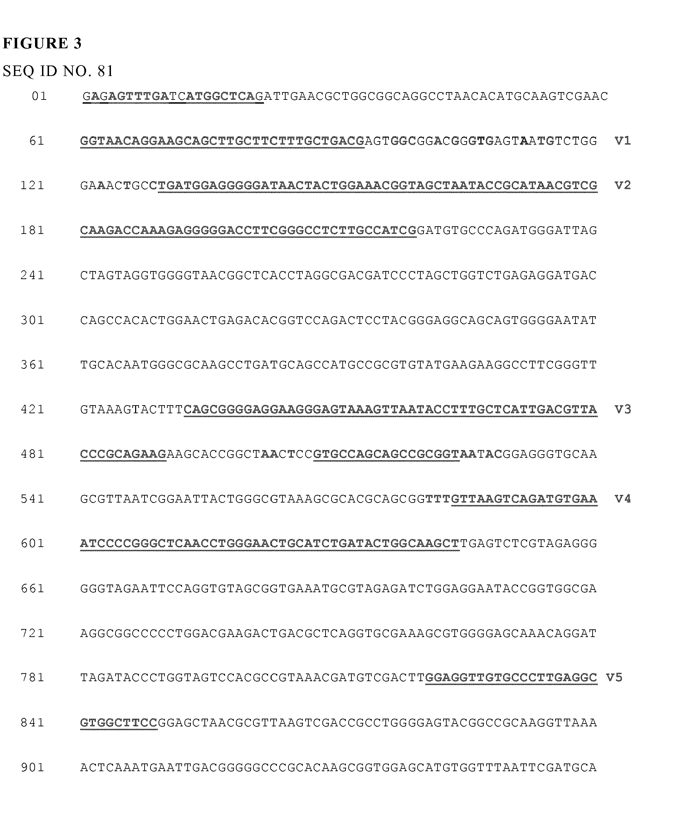

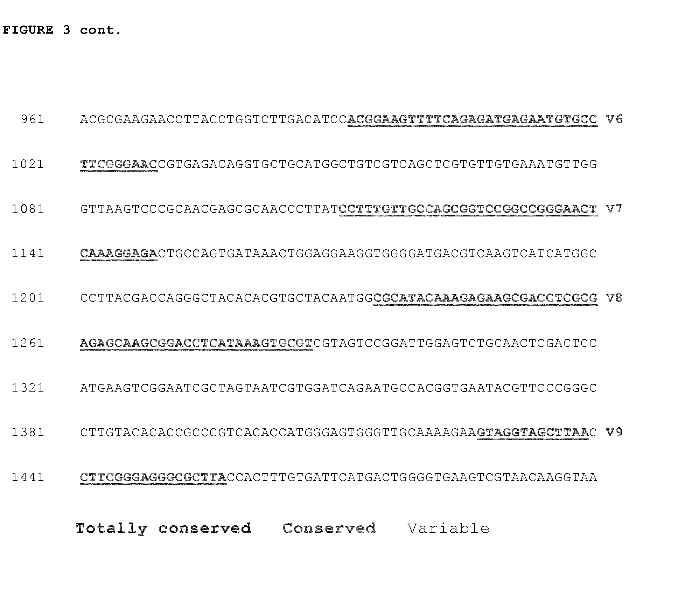

Given the small number of base pairs contained between V1 and V2 and their low level of conservation, the forward primer was designed upstream of V1 (Figure 3).

The presence of polymorphisms in the selected primers might cause a failure to detect some bacterial species and consequently lead to an incomplete picture of the microbiote composition characterization.

For this purpose, different "in sillico" analysis have been driven (data not shown) and have led to the degeneration of 2 bases in the 16S forward primer (figure 3).

The final oligonucleotide design included 454 Life Sciences A or B sequencing titanium adaptors (Roche Diagnostics, Vilvoorde, Belgium) and key sequence fused to the 5' end of the specific target primers. The multiplex identifiers (MIDs) are only included along with the A adaptors.

As seen in Figure 3, two bases has been degenerated due to observed polymorphism across genera (results not shown)

### 16S gene library construction and pyrosequensing

The bacterial V1-V3 regions of the 16S rRNA gene were amplified by PCR using a homemade fusion primer set leading to an amplicon of 625 bp in length. For the GS Junior Titanium chemistry, the forward primer (5' - CCTATCCCCTGTGTGCCTTGGCAGTCTCAGGAGAGTTTGATYMTGGCTCAG -3') (SEQ ID NO. 82) contain the B adaptor, a key sequence (TCAG) and our forward conserved bacterial primer (Roche, Basel, Switzerland). The reverse primer (5'-CCATCTCATCCCTGCGTGTCTCCGACTCAGNNNNNNNNNNTACCGCGGCTGCTG GCAC -3') (SEQ ID NO 83) contain the A adaptor, a key sequence (TCAG), ten bases barcodes (Ns) to distinguish samples and our reverse conserved bacterial primer.

The amplification mix contained 5 U of FastStart high fidelity polymerase (Roche Diagnostics, Vilvoorde, Belgium), 1x enzyme reaction buffer, 200 µM dNTPs (Eurogentec, Liège, Belgium), 0.2 µM of each fusionprimer and genomic DNA in a volume of 25 µl.

The PCR conditions are as follows: 4 min at 94 °C, 25 cycles of denaturation (15 sec at 94 °C), annealing (45 s at 56 °C) and extension (60 s at 72 °C), followed by a final elongation (8 min at 72 °C). These amplifications were performed on an Ep Master system gradient apparatus (Eppendorf, Hamburg, Germany).

The PCR products of 625 nucleotides are checked by gel electrophoresis and purified using the AMPure kit (Agencourt Bioscience Corporation, Beverly, USA) to remove amplicons shorter than 100 bp. Equal amounts of each of the PCR products are pooled and subsequently amplified by emulsion PCR before sequencing. Pyrosequencing is then performed with the Roche 454 GS Junior Sequencer (Roche, Basel, Switzerland) using the massively parallel pyrosequencing protocol.

### Bioinformatic analysis

Image and data processing for amplicon sequencing are performed using the Genome Sequencer FLX System Software Package 2.3 (Roche, Basel, Switzerland). The Mothur program is used for the first part of the sequences analysis. Sequences with a length under 450 bases are discarded. The sequences with ambiguous bases or homopolymers longer than ten nucleotides and with an average quality score lower than 25 are removed along with tag and primer sequences. Sequences from multiplexed samples are assigned based on the presence of the unique barcodes assigned to each sample. The sequences that pass the quality check are aligned to the SILVA alignment database. Chimeric sequences are detected using the chimera-slayer command included in the Mothur package, and the potential chimeras are removed. The sequences are preclustered to reduce false operational taxonomic units produced by erroneous sequences. A distance matrix is prepared, (distance= 0.01) and the sequences are clustered to operational taxonomic units (OTUs) using the average neighbour algorithm. An 80% confidence threshold is required for genus level assignment with the SILVA Database. OTU representing less than 0.01% of the total number of reads are considered as artifacts and discarded.

In a second phase, the representative sequences of each OTU are compared to the NCBI 16S microbial database for example using the Basic Local Alignment Search Tool (BLASTN). The BLAST assignments for each OTU are assigned if the BLASTN default score is above 800. The genus assignations obtained by Mothur and by BLAST are compared and noted as unclassified in a case of mismatch. In cases of several hits with a score above 800, the first hit with the highest score is fostered to give the bacterial species, and 5 following hits with a decreasing score are given for complementary information on the BLAST results.

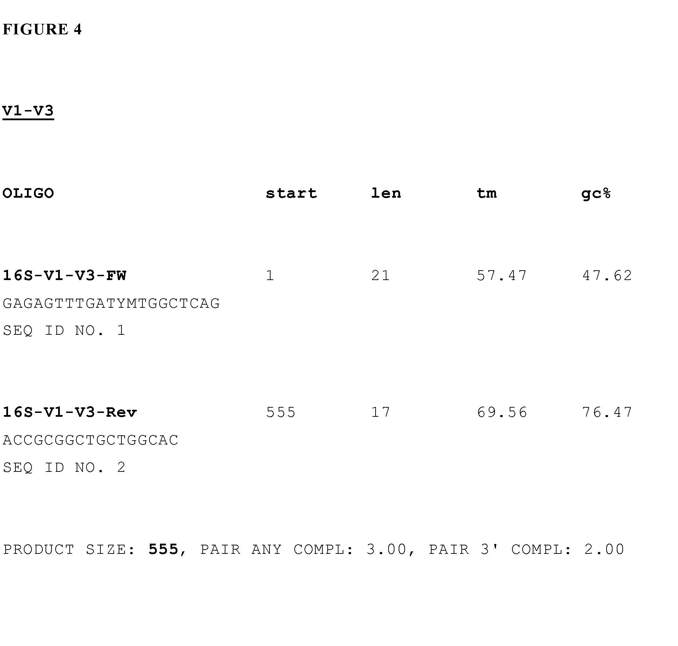

Finally, conversion and normalization of data are carried out. The number of sequences from each OTU is used to compute percentages. The percentage of each OTU is finally converted as a proportion of the total viable count obtained by classical microbiological analysis. Figure 4 gives the flow diagram of the metagenomic analysis of any sample.

The complete description of the bioinformatic homemade pipeline is described in annexe 1 which is herein incorporated in its entirety.

### Semi-quantitative metagenomic analysis

Conversion of sequence proportions into real colony forming units is of great importance especially for the food sector. For a microorganism to induce organoleptic disorders, its average concentration has to reach minimum 10^{5_6} CFU per gram of sample. Indeed, saying that a spoilage organism represents even 90 percent of the observed bacteria does not mean anything if just a low amount of total flora is present in the product. Normalization of the sequences numbers in a log CFU estimation is therefore important.

Microbiological analyses are performed using a classic microbiological approach for the determination of the total bacterial count and, in some extent, the enumeration of bacterial sub-populations.

Ten-fold dilution series of the homogenized samples are prepared in peptone water and plated onto the corresponding medium. Total bacterial count is measured by counting the colony forming units (CFUs) present on plate count agar (PCA) (Oxoid, Dardilly, France) after 48 h of incubation at 30 °C. The results are expressed in logarithms of CFU per gram of sample.

The percentage of each OTU might then be converted as a proportion of the total viable count obtained by classical microbiological analysis.

As metagenomic analysis is a non-cultured based method, DNA derived from dead bacteria is also sequenced. Sequences normalization on the total growing bacterial count is introducing an underestimation. To counteract this bias, qPCR targeting diverse taxa may be provided.

### Discrimination between dead or alive bacteria

Metagenomic analyses have revolutionized microbial detection. However the obstacle for DNA-based techniques is that DNA in the environment can be very stable and can persist for extended periods of time (days to weeks) after cell death. As DNA from dead or alive microorganisms is PCR amplified in the same way, this will lead to an overestimation of microbial targets. One of the prerequisites of making ecological conclusions derived from genetic fingerprints is that bacterial community profiles reflect the live portion of the sample of interest.

Sample treatment with Propidium monoazide (PMA), a DNA intercalating agent, prior to DNA extraction successfully avoids amplification of targets from dead microorganisms. Propidium monoazide is a membrane-impermeant dye that selectively penetrates cells with compromised membranes, which can be considered dead. Once inside the cells, PMA intercalates into the DNA and can be covalently cross-linked to it after exposure to strong visible light. This subsequent complex strongly inhibits further PCR amplification. By using PCR after PMA treatment, the analysis of bacterial communities can theoretically be limited to cells with intact cell membranes.

Twenty-five gram of food sample are homogenized for 1 minute in 225 ml in a tempo bag (bioMérieux Basingstoke, England, ref 80015) with sterile physiologic water using a stomacher apparatus. 1.5 ml of the suspension are then centrifuge. After discarding the supernatant, the pellet is exposed for 10 minutes to PMA treatment (50 µM) in a dark room.

After a 5 minutes light exposure, the solution is centrifuge again and the pellet is directly mixed with the lysis solution prior to DNA extraction using DNeasy Blood & Tissue Kit (QIAGEN, Crawley, UK).

### Elimination of plastid and mitochondrial DNA co-amplification.

In case of prepared food samples containing spices, herbs, fungi, vegetables or fruits; whatever the extraction method used to isolate DNA from these their bacterial communities, the extracted microbial DNA is generally contaminated with DNA of plant origin, in particular chloroplasts and mitochondria. The same problem will occur for any 16S metagenomic analysis aiming to learn about plant-associates microbiotes.

Since the chloroplast 16S and mitochondrial 18S rRNA genes share high sequence similarity with bacterial 16S rRNA sequences contamination with plant DNA poses a serious challenge for the application of PCR-based methods to profile and quantify bacterial populations in plant environments.

Without specific treatment, it is not rare to sequence more than 95 percents of mitochondrial and chloroplastic DNA instead of the bacterial one that is actually targeted, which is quite irrelevant, and undesired considering the price of a metagenome analysis.

PNA (Peptide Nucleic Acid), an artificially created DNA analogue, was invented by Drs. Nielsen, Egholm, Berg, and Buchardt in 1991. The phosphate ribose ring of DNA was replaced with the polyamide backbone in PNA. Despite a radical structural change, PNA is capable of sequence-specific binding in a helix form to its complementary DNA or RNA sequence. Due to its superior binding affinity and chemical/biological stability, PNA has been widely applied in the field of biology (www.panagen.com).

PNA PCR clamping technique was developed very early to detect SNP in low abundance. For example, a few mutant cancerous cells may be among healthy cells in a tissue biopsy. PNA clamp complementary to wild type sequence hybridizes specifically with the wild type and blocks its PCR amplification while allowing amplification of mutant sequence of imperfect match. A single base mismatch is enough to discriminate amplification of mutant type from wild type.

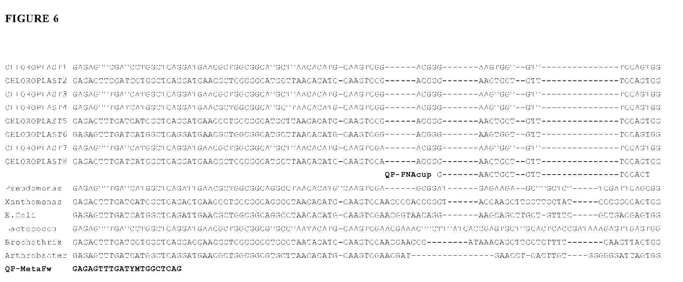

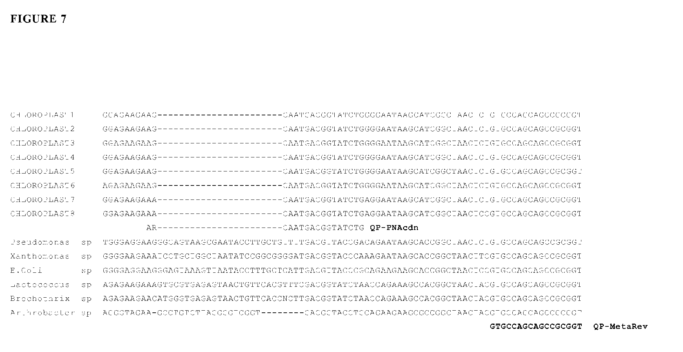

Two PNA clamps have been developed to block the co-amplification of the chloroplastic DNA (Figures 6 and 7).

A third PNA clamp has been developed to block the co-amplification of the mitochondrial DNA (Figure 8).

The use of those 3 PNA clamps in the PCR mix allow to mainly amplify the desired bacterial 16S DNA for the microbiome profiling assay.

### Elimination of specific undesired over-represented taxa.

In some cases, the bacterial diversity might be hidden by an over represented taxa.

One example is the presence of a huge amount of *Lactococcus* species in any cheese as these taxa are extensively used as starter in the production of buttermilk and cheese. Specific PNA clamping probes have been designed to avoid the amplification of any bacteria belonging to the *Lactococcus* taxa in order to give emphasis on the environmental contaminating flora or on any other species contributing to the specific texture, flavour or odour of the cheese.

On the same principal of the mitochondrial or plastid design, we founded a PNA sequence between our metagenomic V1-V3 primers. The PNA probe is characterized by a sequence conserved for example across the *Lactococcus* taxa but showing at least one point mutation compare to the other bacterial classes frequently observed in cheese products.

The alignment used for the lactococcus PNA probe is represented in Figure 9.

### Results

### Descriptive metagenomic analysis

The present invention might be used for a complete description of the total flora of any sample (food, beverage, stools, any biological sample, soil, water or any environment sample)

As an example of a descriptive metagenomic analysis, we analyse a set of steak tartare samples. Steak tartare is indeed a popular meat dish in Belgium and other European countries. This meat preparation, due to its raw nature, is highly sensitive to bacterial spoilage. A better understanding of the bacterial content of this product will thus be insightful to control the risk of spoilage. Metagenomics targeted on the 16S ribosomal DNA has appeared as a powerful tool to study bacterial composition of food samples. The aim of this study is to identify the bacterial populations of steak tartare from different origins along their shelf life and determine the main spoilage bacterial species.

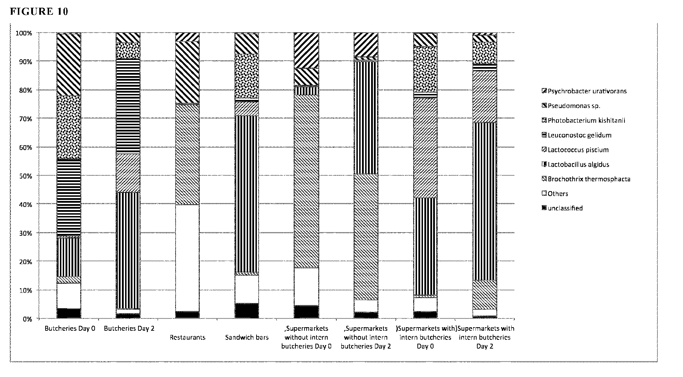

Results of the metagenomic analysis on steak tartare are shown in Figure 10. Each lane represents 100% of the bacterial composition of one sample. Each colour corresponds to a certain amount of sequences assign to a certain taxa.

A total of 32 dominant bacterial species were identified in the steak tartare samples by the metagenomic analysis. *Brochothrix thermosphacta, Lactobacillus algidus, Lactococcus piscium, Leuconostoc gelidum, Photobacterium kishitanii, Pseudomonas sp.* and *Psychrobacter urativorans* are the most predominant bacterial species in the samples and represent 89% of the detected bacterial species.

*Brochothrix thermosphacta and Lactobacillus algidus,* represent 50% of all the detected species. *Brochotrix thermosphacta* is detected mostly in samples from restaurants (2,8-93,9 %; n=4/6) and supermarkets without intern butcheries (0,3-83,2 %; n=8/8). *Lactobacillus algidus* is present in each category but in low proportion for restaurants (2,3-5,5 %, n=3/6). *Pseudomonas sp, Brochotrix thermosphacta* or *Leuconostoc gelidum* are present in high proportion in some samples at Day 0. These bacteria are well known to spoil meat product and samples that contain these bacteria in high proportion are more susceptible to spoil rapidly. Some samples (data not shown) contain a high level of *Streptococcus* or *Enterobacter* resulting probably of cross contamination during the meat process.

A food with a concentration above 6 log CFU/g is generally considered as spoiled. However, the results indicate that some samples have a concentration around this theoretical limit and contains a high proportion of bacteria which are not considered as spoilage bacteria *like Lactobacillus algidus.*

The metagenomic analysis is a powerful tool to identify and to measure the relative proportions of dominant bacterial species in the steak tartare as any other food or non food samples.

Some bacterial species could be indicators of the meat quality. Identification of some bacterial species over time gives information on the evolution of the spoilage process.

Therefore, metagenomic analysis could be an additional tool to control and manage the meat quality as well as the risk of spoilage.

### Semi-quantitative metagenomic analysis

The main drawbacks of the metagenomic approach is the relativity of the proportions of the bacterial populations. The following example shows that linking these proportions to a validated estimated bacterial count is an actual tool to accurately qualify the quality of a food sample.

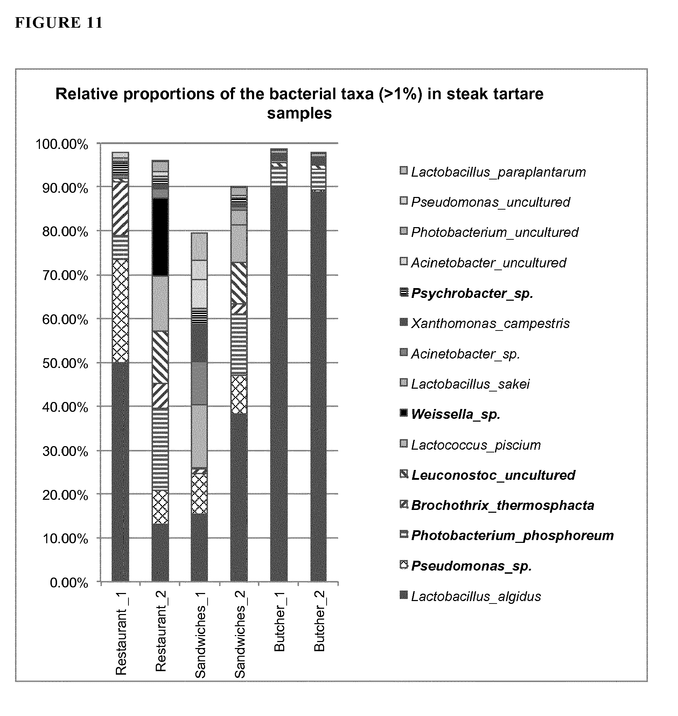

The figure 11 shows the relative proportions of bacteria found in several samples of steak tartare preparation obtained from 2 restaurants, 2 from sandwich vendors and 2 from butcheries. The metagenomic sequencing and analysis followed the same protocol as described above.

The samples are clearly populated with various populations with a leading domination of Lactic Acid Bacteria (LAB). The first taxon is *Lactobacillus algidus,* which is a common bacterium frequently found in cold stored beef meat. This bacterium is homofermentar and not known for any spoilage activity. Another LAB is *Lactococcus piscium* which again is a non-spoiling bacteria.

Other populations observed however are known to possess spoiling activity when their population is high enough. This is the case for *Leuconostoc,* sp., *Weissella* sp., *Photobacterium phosphoreum* and *Brochotrhix thermosphacta.*

We would like to focus on 2 samples: Restaurant-2 and sandwich-2. These samples possess the highest levels of contamination by *Leuconostoc, Weissella* and *Photobacterium* in terms of relative proportions.

Based on these observations, one could argue that both samples are not of good quality because of the presence of spoilage bacteria (*Leuconostoc* giving a cheesy and slimy flavor on the meat). But, in fact these relative proportions don't reflect the true level of bacterial contamination.

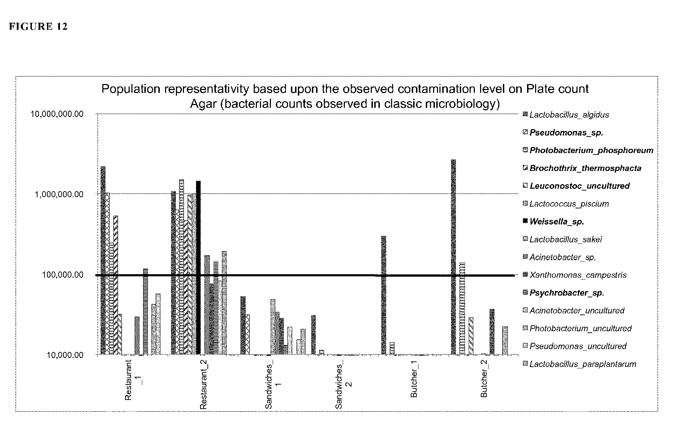

In order to visualize the results from the point of view of a food microbiologist, we convert the OTU populations into estimated log CFUs, taking the following as an arbitrary statement: that the proportion of an OTU to the entire OTU population was related to the same proportion of log CFUs to the log of the total plate count (Figure 12). As we chose matrices actively invaded by bacteria, these were considered as active and thus correctly represented by the colonies grown on plates. This is not a canonical choice but it allows the selection of OTU populations with a size of over 100,000 cells; a threshold above which is said to lead to spoilage for most altering bacteria in foodstuff at the shelf life limit.

In the sample restaurant-2, several spoilage bacteria are present with a population level above 1,000,000 cells per gram. Such level clearly has a noticeable sensory effect. On the contrary, the sample Sandwich-2 is in fact lowly contaminated. As the total flora count of mesophilic bacteria is below 10,000 cells per gram, it doesn't appear on the graph. Thus the spoilage population, event if they are present, will not have a sensory effect at the time of sampling.

This combination allows us to clearly see the true impact of the bacteria present.

### Discrimination between dead or alive bacteria

Metagenomics is an extremely valuable tool for probiotics quality control. The methodology of the invention enables the validation of the determined bacterial and/or yeast content of the product. As described above, by adding a PMA treatment to the sample prior to the DNA extraction, the viability of the determined micro-organisms might be assessed.

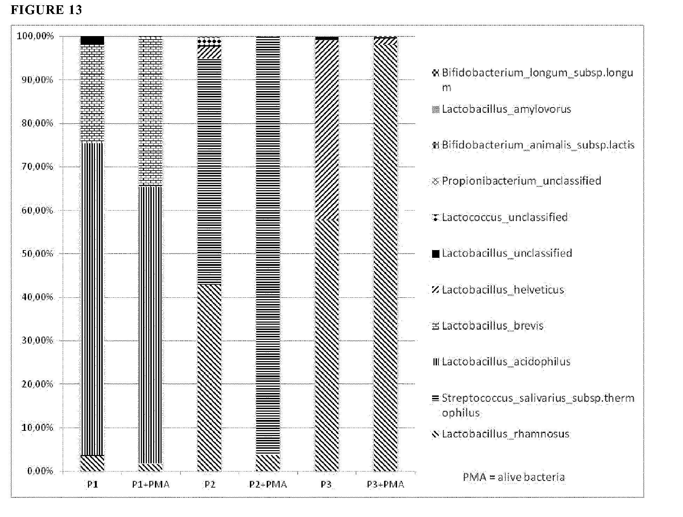

As seen in figure 13, the proportions of some bacterial groups are modified by the PMA treatment. By comparison between a sample PMA treated (Px^{PMA}) or not (Px), one can see that the total amount of certain species is decreasing after a PMA treatment meaning that the analysis conclude positively about the presence of such a bacterial group in the probiotic but underline the fact that the viability of this group is not well conserved. Indeed, the decrease of proportion indicate that part of those bacteria are actually already dead which is not interesting as the effect of a probiotic intake will be due to the gainful activity of the viable bacteria.

### Elimination of plastid and mitochondrial DNA co-amplification.

Since the chloroplast 16S and mitochondrial 18S rRNA genes share high sequence similarity with bacterial 16S rRNA sequences, contamination with plant DNA poses a serious challenge for the application of PCR-based methods to profile and quantify bacterial populations in plant environments. Microbiome profiling of a food product mixed with herbs, spices or any vegetable is really challenging without specific treatment avoiding the amplification of chloroplastic or/of mitochondrial DNA. Without this kind of treatment, the proportion of those "contaminating" DNA could indeed represent in some cases until 99 percents of the reads obtained by the high-throughput sequencing. Considering the cost of an analysis, sequencing 9900 sequences out of 10000 coming out of spices when trying to analyze the bacterial diversity of the product is definitely not what is expected.

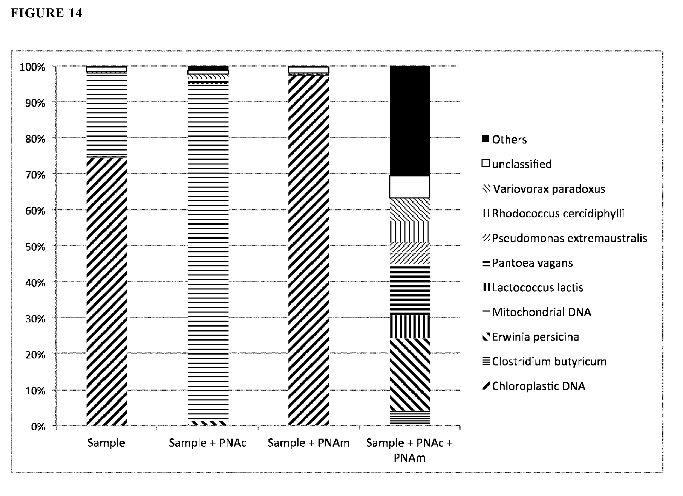

We analyze such a product at the date of production normally meaning with a low amount of bacterial flora. Without any treatment, the majority of the DNA sequences were coming from chloroplastic DNA. Based on the bacterial and chloroplastic DNA alignment described earlier, 2 PNA clamping probes were design to block the amplification of any chloroplastic DNA during the 16S specific PCR. Using those probes, no DNA was amplified from chloroplast but the majority of the produced sequences were from mitochondrial DNA. On the same bases, we then design a PNA clamping probe directed against the amplification of any mitochondrial DNA. By combining the 3 PNA clamping probes, we could finally observe the bacterial diversity of the product (figure 14)

Without those probes, the bacterial diversity of many food or agricultural samples are just impossible to explore.

### Elimination of specific over-represented taxa.

In fermented food, the use of a starter to induce the fermentation has often as a result that those microorganisms that are part of the recipe characterize a major part of the final ecosystem of the product.

In case of quality problem leading to a non-conform product, it is really important to be able to identify the microbial diversity often hidden by the major starter microorganisms. As for vegetable products, the invention is using a PNA clamping probe to block the amplification of those "contaminant" flora to be able to see the diversity behind.

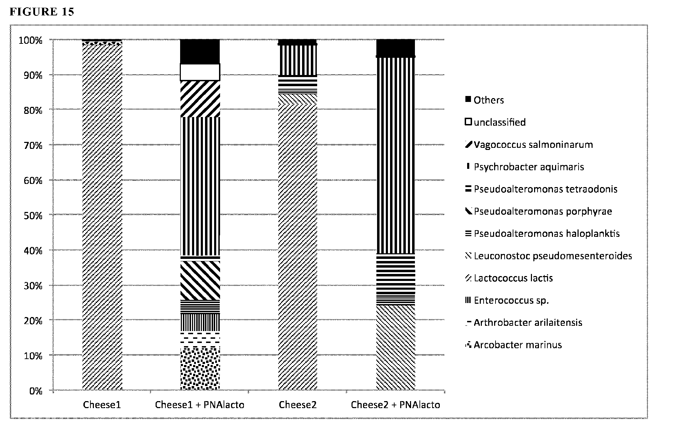

Figure 15 represents the results of a metagenomic analysis of two different cheese cords with and without PNA probe designed against the amplification the lactococcus taxa representing the major proportion of their microbial flora.

*Lactococcus* lactis is introduced thought the starter to initiate the cheese fermentation. In both cheeses, the bacterial profile is dominated by the *lactococcus* genera preventing the underlying diversity to be observed. As can be clearly seen in figure 15, the use of the PNA clamp designed to block the amplification of any *Lactococcus* species has a huge effect on the observed bacterial profile. When PNALactococcus is used in the metagenomic protocols, the *Lactococcus* genus clearly disappeared in favour of the underlying diversity.

### Conclusion

Metagenomic analysis offers a new tool for identifying microorganisms present in perishable food and their evolution depending of environmental conditions. The information available through metagenomics analysis provides a clear picture of the microbial community. Microbiological ecology studies have shown that the bacterial world of foods are much more diverse than the cultivable group of bacteria studied by culture media. For the metagenomic analysis, 3 to 5 thousand sequences appeared largely sufficient to identify and to characterize the spoiling microflora, though that might change depending on the objective and the budget of a particular study. Many food manufacturers, government agencies, retailers, distribution quality laboratories and researchers use general culture media without precisely knowing the bacterial communities inside the food. Compared to culture based methods on selective media and previous independent culture techniques, metagenomic analysis combined with the enumeration of psychrotrophic flora gives more valuable information, and its use should be considered as a technique for quality control, for accurately determining shelf life or for developing new food products.

The invention is not limited by the examples or figures provided herein. The skilled person is able to conceive of alternatives for the various sequences, tools and methods mentioned in this specification. The invention is limited only by the scope of the claims.

## Claims

1. A method of metagenomic analysis of a food or stool sample to detect and quantitate microorganisms present in said sample, the analysis comprising amplification of the V2-V3 region of bacterial 16S rRNA in said sample.

2. A method of claim 1 wherein the V1-V3 region of bacterial 16S rRNA is amplified.

3. A method of claim 1 or claim 2 wherein said analysis can detect and distinguish bacteria of the families: *Acetobacteraceae, Aeromonadaceae, Bacteroidaceae, Burkholderiaceae, Carnobacteriaceae, Clostridiaceae, Comamonadaceae, Enterobacteriaceae, Enterococcaceae, Flavobacteriaceae, Lachnospiraceae, Lactobaillaceae, Leuconostocaceae, Listeriaceae, Moraxellaceae, Neisseriaceae, Pseudoalteromonadaceae, Pseudomonadaceae, Shewanellaceae, Streptococcaceae, Vibrionaceae* and *Xanthomonadaecea.*

4. A method of any preceding claim wherein said analysis can detect and distinguish bacteria of the genuses: *Acetobacteraceae Acetobacter, Acetobacteraceae Gluconacetobacter, Aeromonadaceae Aeromonas, Bacteroidaceae Bacteroides, Burkholderiaceae Burkholderia, Carnobacteriaceae Carnobacterium, Clostridiaceae Clostridium, Comamonadaceae Comamonas, Comamonadaceae Delftia, Enterobacteriaceae Escherichia, Enterobacteriaceae Pantoea, Enterobacteriaceae Serratia, Enterococcaceae Enterococcus, Chryseobacterium, Flavobacteriaceae Flavobacterium, Flavobacteriaceae Myroides, Lachnospiraceae Butyrivibrio, Lactobaillaceae Lactobacillus, Lactobacillaceae Pediococcus, Leuconostocaceae Leuconostoc, Leuconostocaceae Weissella, Listeriaceae Listeria, Moraxellaceae Acinetobacter, Moraxellaceae Psychrobacter, Neisseriaceae Alysiella, Pseudoalteromonadaceae Pesudoalteromonas, Pseudomonadaceae Pseudomonas, Shewanellaceae Shewanella, Streptococcaceae Lactococcus, Streptococcaceae Streptococcus, Vibrionaceae Aliivibrio, Vibrionaceae Photobacterium, Vibrionaceae Vibrio* and *Xanthomonadaecea Xanthomonas.*

5. A method of any preceding claim wherein the percentage of microorganisms represented by at least one operational taxonomic unit (OTU) is determined.

6. A method of claim 5 further comprising determining the colony forming units count (CFU) of the sample and normalising the percentage of organisms represented by said OTU to a proportion of the total viable CFU count of the sample.

7. A method of any preceding claim wherein the said metagenomic analysis is followed by taxa specific qPCR.

8. A method of any preceding claim wherein the metagenomic analysis is conducted using a primer comprising the sequence of SEQ ID NO. 1.

9. A method of any preceding claim wherein the metagenomic analysis is conducted using a primer comprising the sequence of SEQ ID NO. 2.

10. A method of any preceding claim wherein the metagenomic analysis is conducted using a pair of primers comprising the sequences of SEQ ID NO 1 and SEQ ID NO. 2.

11. A method of any of claims 8 to 10 wherein said primers consist of the sequences of SEQ ID NO. 1 or SEQ ID NO. 2 respectively.

12. A method of any of claims 8 to 11 wherein said primer is modified by the addition of an adaptor, key sequence, tag or universal sequence, optionally having the sequences of SEQ ID NO.s 82 and 83 respectively.

13. The use of Peptide Nucleic Acid (PNA) clamping in a method of metagenomic analysis of a sample, to reduce amplification of at least one of: i) chloroplast DNA present in the sample; ii) mitochondrial DNA present in the sample; or iii) DNA from one or more selected microorganisms that may be present in the sample.

14. A use of claim 13 wherein said PNA clamping comprises conducting metagenomic analysis in the presence of a PNA oligonucleotide comprises a sequence derived from a chloroplast 16S rRNA gene, preferably a sequence common to a plurality of chloroplast 16S rRNA genes.

15. A use of claim 14 wherein said PNA oligonucleotide comprises a sequence common to at least two of, and preferably all of, the chloroplast 16S rRNA sequences of SEQ ID NOs 3 to 10, or common to at least two of, and preferably all of, the chloroplast 16S rRNA sequences of SEQ ID NOs 18 to 25.

16. A use of claim 14 or claim 15 wherein said PNA olignucleotide has a sequence not found in the bacterial 16S rRNA sequences of at least one of, and preferably all of, SEQ ID NOs 11 to 16, or has a sequence not found in the bacterial 16S rRNA sequences of at least one of, and preferably all of, SEQ ID NOs 26 to 31.

17. A use of any of claims 14 to 16 wherein said PNA oligonucleotide comprises the sequence of SEQ ID NO. 17, optionally wherein said PNA oligonucleotide consists of the sequence of SEQ ID NO. 17.

18. A use of any of claims 14 to 16 wherein said PNA oligonucleotide comprises the sequence of SEQ ID NO. 32, optionally wherein said PNA oligonucleotide consists of the sequence of SEQ ID NO. 32.

19. A use of claim 13 wherein said PNA clamping comprises conducting metagenomic analysis in the presence of a PNA oligonucleotide comprises a sequence derived from a mitochondrial 18S rRNA gene, preferably a sequence common to a plurality of mitochondrial 18S rRNA genes.

20. A use of claim 19 wherein said PNA oligonucleotide comprises a sequence common to at least two of, and preferably all of, the mitochondrial 18S rRNA sequences of SEQ ID NOs 33 to 38.

21. A use of claim 19 or claim 20 wherein said PNA olignucleotide has a sequence not found in the chloroplast 16S rRNA sequences, or the bacterial 16S rRNA sequences of at least one of, and preferably all of, SEQ ID NOs 39 to 47.

22. A use of any of claims 19 to 21 wherein said PNA oligonucleotide comprises the sequence of SEQ ID NO. 48, optionally wherein said PNA oligonucleotide consists of the sequence of SEQ ID NO. 48.

23. A use of claim 13 wherein said PNA clamping comprises conducting metagenomic analysis in the presence of a PNA oligonucleotide comprises a sequence derived from a bacterial 16S rRNA gene, preferably a sequence common to a plurality of bacterial 16S rRNA genes.

24. A use of claim 23 wherein said PNA oligonucleotide comprises a sequence common to at least two of, and preferably all of, the bacterial 16S rRNA sequences of SEQ ID NOs 49 to 61.

25. A use of claim 23 or claim 24 wherein said PNA olignucleotide has a sequence not found in the bacterial 16S rRNA sequences of at least one of, and preferably all of, SEQ ID NOs 62 to 79.

26. A use of any of claims 23 to 25 wherein said PNA oligonucleotide comprises the sequence of SEQ ID NO. 80, optionally wherein said PNA oligonucleotide consists of the sequence of SEQ ID NO. 80

27. A use of any of claims 13 to 26 wherein the metagenomic analysis is characterised as defined in any of claims 1 to 12.

28. A method of metagenomic analysis of a sample, wherein propidium monoazide (PMA) is added to the sample prior to DNA extraction in order to reduce the subsequent amplification of DNA from non-viable microorganisms present in the sample.

29. A method of claim 28 wherein the metagenomic analysis is characterised as defined in any of claims 1 to 12, and/or includes a use of any of claims 13 to 27.

30. A kit for metagenomic analysis of a sample comprising one or both primers comprising the sequence of SEQ ID NO. 1 and/or SEQ ID NO. 2, preferably wherein the primers consist of the sequences of SEQ ID NO. 1 and/or SEQ ID NO. 2.

31. A kit of any claim 30 wherein the primer(s) are modified by the addition of an adaptor, key sequence, tag or universal sequence, optionally having the sequences of SEQ ID NO.s 82 and 83 respectively.

32. A kit of claim 30 or claim 31 further comprising a PNA oligonucleotide comprising a sequence of any of SEQ ID NOs. 17, 32, 48 or 80, preferably wherein the primer consists of the sequence of any of SEQ ID NOs. 17, 32, 48 or 80.

33. A kit of claim 32 comprising a set of primers including any two, three or four PNA oligonucleotides comprising or consisting of the sequence of SEQ ID NO.s 17, 32, 48 or 80.

34. A kit of any of claims 30 to 33 further comprising propidium monoazide (PMA).

35. A kit of any of claims 30 to 34 further comprising a reagent for metagenomic analysis of a sample, e.g. a food or stool sample.
